(19) Europäisches Patentamt
European Patent Office
Office européen des brevets

(11) **EP 2 665 414 B1**

(12) **FASCICULE DE BREVET EUROPEEN**

(45) Date de publication et mention de la délivrance du brevet:
**24.07.2019 Bulletin 2019/30**

(21) Numéro de dépôt: **12700292.1**

(22) Date de dépôt: **17.01.2012**

(51) Int Cl.:
*A61B 5/0488* (2006.01)    *A61B 5/103* (2006.01)
*A61B 5/11* (2006.01)

(86) Numéro de dépôt international:
**PCT/EP2012/050649**

(87) Numéro de publication internationale:
**WO 2012/098121 (26.07.2012 Gazette 2012/30)**

(54) **MESURE DE LA SPASTICITE**

SPASTIZITÄTSMESSUNG

SPASTICITY MEASURING

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.01.2011 FR 1150361**

(43) Date de publication de la demande:
**27.11.2013 Bulletin 2013/48**

(73) Titulaires:
• **Université Technologie de Compiègne-UTC**
**60203 Compiegne cedex (FR)**
• **Centre National de la Recherche Scientifique (C.N.R.S.)**
**75016 Paris (FR)**

(72) Inventeurs:
• **GAMET, Didier**
**60205 Compiègne Cedex (FR)**
• **BUFFENOIR-BILLET, Kevin**
**44000 Nantes (FR)**
• **PEROT, Chantal**
**60205 Compiègne Cedex (FR)**

(74) Mandataire: **Regimbeau**
**20, rue de Chazelles**
**75847 Paris Cedex 17 (FR)**

(56) Documents cités:
**WO-A1-2010/121353**    **US-A1- 2007 027 631**
**US-A1- 2008 071 386**    **US-A1- 2008 312 549**

• **CHUNG ET AL: "Separate Quantification of Reflex and Nonreflex Components of Spastic Hypertonia in Chronic Hemiparesis", ARCHIVES OF PHYSICAL MEDICINE AND REHABILITATION, W.B. SAUNDERS, UNITED STATES, vol. 89, no. 4, 25 mars 2008 (2008-03-25), pages 700-710, XP022549777, ISSN: 0003-9993, DOI: 10.1016/J.APMR.2007.09.051**
• **KOBAYASHI T ET AL: "Quantitative measurement of spastic ankle joint stiffness using a manual device: A preliminary study", JOURNAL OF BIOMECHANICS, PERGAMON PRESS, NEW YORK, NY, US, vol. 43, no. 9, 18 juin 2010 (2010-06-18), pages 1831-1834, XP027075211, ISSN: 0021-9290, DOI: 10.1016/J.JBIOMECH.2010.02.024 [extrait le 2010-02-26]**

EP 2 665 414 B1

**Description**

L'invention concerne le domaine médical et plus particulièrement le domaine de la mesure de la spasticité.

On rappelle que la spasticité est une exagération du réflexe myotatique. Plus précisément, elle est une contraction réflexe exagérée d'un muscle en réaction à son étirement. La spasticité peut-être uniforme sur tout le corps mais elle est le plus souvent localisée sur les membres inférieurs (diplégie spastique) ou sur un hémicorps.

Pour déterminer de manière non invasive la présence d'une spasticité et pouvoir l'évaluer, il existe une procédure appelée « manoeuvre d'Ashworth » (Ashworth B., Preliminary trial of carisoprodol in multiple sclerosis, Practionner 1964 ; 192:540-542), manoeuvre qui caractérise le degré de spasticité. Cette manoeuvre consiste à fléchir passivement l'articulation de la cheville du patient qui déclenche chez le sujet spastique un réflexe d'étirement caractérisé par une brève extension de l'articulation. Celle-ci est perçue par le praticien et permet de qualifier l'ampleur de la spasticité sur une échelle spécifique appelée «Echelle d'Ashworth Modifiée» allant de 0 à 4:

◦ 0 : Tonus musculaire normal,
◦ 1 : Augmentation discrète du tonus musculaire se manifestant par un ressaut suivi d'un relâchement ou par une résistance minime en fin de mouvement,
◦ 1+ : Augmentation discrète du tonus musculaire se manifestant par un ressaut suivi d'une résistance minime perçue sur moins de la moitié de l'amplitude articulaire,
◦ 2 : Augmentation plus marquée du tonus musculaire touchant la majeure partie de l'amplitude articulaire, l'articulation pouvant être mobilisée facilement,
◦ 3 : Augmentation importante du tonus musculaire rendant la mobilisation passive difficile,
◦ 4 : l'articulation concerné est fixée en flexion ou en extension (abduction ou adduction).

Cette technique permet donc une évaluation de la spasticité. Toutefois, cette manoeuvre passe par l'appréciation tactile du praticien ; elle reste donc purement qualitative et très subjective.

Afin de permettre une mesure plus objective, on connaît des myomètres permettant de mesurer le tonus musculaire. Toutefois de nombreuses critiques ont été émises à l'encontre de ces dispositifs en ce qu'ils ne permettent pas, en soi, de mesurer la spasticité; en effet, celle-ci n'est pas liée uniquement au tonus musculaire. En outre, ces dispositifs sont difficilement manipulables durant la manouvre d'Ashworth puisque cette dernière nécessite les deux mains du praticien.

La demande internationale WO 2010/121353 décrit un appareil portable de mesure de la spasticité, qui repose sur une théorie canadienne qui qualifie la spasticité à l'aide de l'occurrence de l'activité électromyographique et de la vitesse du mouvement) à partir d'un angle remarquable dit « Dynamic Strech Reflex Threshold ». Ce dispositif permet ainsi d'identifier une atteinte de spasticité chez un patient, mais en aucun cas de la qualifier sur l'échelle d'Ashworth

Les documents:

CHUNG ET AL: "Separate Quantification of Reflex and Nonreflex Components of Spastic Hypertonia in Chronic Hemiparesis", ARCHIVES OF PHYSICAL MEDICINE AND REHABILITATION, W.B. SAUNDERS, UNITED STATES, vol. 89, no. 4, 25 mars 2008 (2008-03-25), pages 700-710,
KOBAYASHI T ET AL: "Quantitative measurement of spastic ankle joint stiffness using a manual device: A preliminary study", JOURNAL OF BIOMECHANICS, PERGAMON PRESS, NEW YORK, NY, US, vol. 43, no. 9, 18 juin 2010 (2010-06-18), pages 1831-1834,
US 2008/071386 A1,
US 2008/312549 A1, et
US 2007/027631 A1 divulguent aussi des dispositifs de mesure de la spasticité.

En conséquence, un but de la présente invention est de fournir un dispositif de mesure de la spasticité d'un sujet permettant de déterminer un score de spasticité de manière objective afin d'affiner de façon sensible le diagnostic d'atteinte.

Un autre but de la présente invention est de fournir un dispositif de mesure de la spasticité qui soit facilement manipulable par le praticien.

A cet égard, l'invention concerne dispositif de mesure de la spasticité d'une articulation (A) d'un sujet, l'articulation étant située entre un premier membre (M1) et d'un deuxième membre (M2), le dispositif étant caractérisé en ce qu'il comprend :

une première partie destinée à recevoir tout ou partie du premier membre (M1) de l'articulation (A),
une deuxième partie destinée à recevoir tout ou partie du second membre (M2) de l'articulation (A), la deuxième partie étant en liaison pivot avec la première partie autour d'un axe de rotation, et

des moyens de détermination d'un score de spasticité à partir de mesures de la réaction de ladite articulation (A) à une flexion exercée sur l'articulation (A), les moyens de détermination comprenant :

un moyen de mesure d'un couple de réaction de l'articulation (A) au niveau de l'axe de rotation,
une unité de traitement de la mesure du couple de réaction de l'articulation (A) au niveau de l'axe de rotation par le moyen de mesure, l'unité de traitement étant destinée à déterminer :

la composante continue de ladite mesure,
la composante alternative de ladite mesure,
un moyen de mesure de l'activité musculaire d'une partie des muscles de l'articulation (A), et
un moyen de mesure de l'angle (G) formé entre les deux parties du dispositif.

Avantageusement, mais facultativement, l'invention comprend au moins l'une des caractéristiques suivantes :

- le moyen de mesure de l'activité musculaire d'une partie des muscles de l'articulation comprend au moins deux électrodes permettant d'établir un électromyogramme de ladite activité musculaire,
- la distance entre les au moins deux électrodes et l'axe de rotation est réglable,
- au moins une des première et deuxième parties du dispositif comprend une surface supérieure et la distance entre ladite surface supérieure et l'axe de rotation est réglable,
- les moyens de détermination comprennent en outre un moyen de transmission de données, préférentiellement un moyen de transmission sans fil,

L'invention concerne un procédé d'utilisation de ce dispositif de mesure, selon la revendication 6. La description fournira plutôt à l'homme du métier une feuille de route adaptée pour implémenter un mode de réalisation exemplaire. Il est entendu que différentes modifications peuvent être réalisées, dans la portée de la matière revendiquée dans la présente, telle qu'exposée dans les revendications annexées.

D'autres caractéristiques, buts et avantages de la présente invention apparaîtront à la lecture de la description détaillée qui va suivre d'un exemple non limitatif de mise en oeuvre, donné au regard des figures annexées sur lesquelles :

- la figure la est une représentation en vue cavalière du dispositif selon une réalisation exemplaire,

- la figure 1b est une représentation schématique en vue de dessus du dispositif selon une réalisation exemplaire,

- la figure 1c est une représentation schématique en vue de côté du dispositif selon une réalisation exemplaire accueillant une articulation d'un sujet,

- la figure 2 est une représentation schématique en vue de côté de la liaison pivot du dispositif selon une réalisation exemplaire,

- la figure 3a est une représentation schématique en vue de côté de la deuxième partie du dispositif selon une réalisation exemplaire,

- la figure 3b est une autre représentation schématique en vue de côté de la deuxième partie du dispositif selon une réalisation exemplaire,

- la figure 3c est une autre représentation schématique en vue de dessus de la deuxième partie du dispositif selon une réalisation exemplaire,

- la figure 3d est une représentation schématique en vue cavalière de l'extension de la deuxième partie du dispositif selon une réalisation exemplaire,

- la figure 3e est une représentation schématique éclatée en vue de côté d'un élément de liaison entre l'élément en plaque et l'extension de la deuxième partie du dispositif selon une réalisation exemplaire,

- la figure 3f est une représentation schématique éclatée en vue de côté d'un autre élément de liaison entre l'élément en plaque et l'extension de la deuxième partie du dispositif selon une réalisation exemplaire,

- la figure 4 est un graphe schématique fonctionnel représentant un procédé d'utilisation du dispositif,

- la figure 5a est une vue de coté du dispositif selon une réalisation exemplaire,

- la figure 5b est une représentation schématique en vue de dessus du dispositif selon une réalisation exemplaire,

- la figure 5c est une représentation schématique éclatée de côté d'un élément de liaison entre la première et la deuxième partie du dispositif selon une réalisation exemplaire,

- la figure 5d est une représentation schématique éclatée de côté d'un élément de liaison entre la première et la deuxième partie du dispositif selon une réalisation exemplaire, intégrant un capteur angulaire,

- la figure 6a représente les signaux bruts obtenus pour un premier patient lors d'un test de manoeuvre lente,

- la figure 6b représente les signaux bruts obtenus pour un premier patient lors d'un test de manoeuvre rapide,

- la figure 6c représente les signaux bruts obtenus pour un deuxième patient lors d'un test,

- la figure 7 représente les signaux traités obtenus pour un troisième patient lors d'un test.

En référence aux figures la à 1c, un dispositif de mesure de la spasticité d'une articulation A, tel qu'une cheville d'un sujet, située entre un premier membre M1 (tel que le pied) et d'un deuxième membre M2 (tel que la partie inférieure de la jambe) comprend :

- une première partie 10 destinée à recevoir tout ou partie du premier membre M1 de l'articulation A du sujet,
- une deuxième partie 12 destinée à recevoir tout ou partie du second membre M2 de l'articulation A du sujet, la deuxième partie 12 étant en liaison pivot avec la première partie 10 autour d'un axe de rotation 14.

Il est à noter que dans la suite du texte, la « liaison pivot » entre la première et la deuxième partie doit être comprise dans un sens large. Bien qu'elle soit décrite ici comme une liaison pivot selon un seul axe, elle peut être facilement généralisable à plusieurs axes de rotation et devenir ainsi une liaison rotule entre le premier et le deuxième membre.

Préférentiellement, la première partie 10 est une plaque ayant une surface supérieure 100 destinée à recevoir le premier membre M1. La première partie 10 a une forme de disque 102 avec une extension rectangulaire 101 (se prolongeant dans le même plan que le disque). La première partie 10 comprend un membre de charnière 103 s'étendant au niveau de l'extension rectangulaire 101 perpendiculairement à la surface 100.

La deuxième partie 12, quant à elle, comprend une plaque 121 ayant une surface supérieure 1211 destinée à recevoir une partie basse du membre M2 et une surface inférieure 1210. La plaque 121 est globalement rectangulaire, les bords pouvant être arrondis. La deuxième partie 12 comprend un membre de charnière 123 s'étendant perpendiculairement à la surface supérieure 1211.

La deuxième partie 12 comprend également une extension 122. Cette extension 122 a une forme de plaque avec une surface supérieure 1221 destiné à recevoir une partie haute du membre M2 et une surface inférieure 1220. L'extension 122 et la plaque 121 sont reliées tel que la surface inférieure 1210 de la plaque 121 et la surface supérieure 1221 de l'extension 122 sont parallèles et en contact l'une avec l'autre. La liaison entre ces deux éléments sera décrite plus en détail ultérieurement. L'extension 122 comprend également un rebord 1222 sur le pourtour d'une extrémité de l'extension 122. Ce rebord définit une paroi concave vers un point situé au dessus de la surface 1221 tel que ce rebord 1222 permet de former un logement destiné à accueillir une partie haute du deuxième membre M2 et plus particulièrement le mollet dans le cas d'une cheville.

Les membres de charnière 103 et 123 (respectivement de la première partie 10 et de la deuxième partie) sont reliés l'une à l'autre tel que la deuxième partie 12 est en liaison pivot avec la première partie 10 autour d'un axe de rotation 14. Cette liaison sera décrite plus en détail par la suite.

Le dispositif comprend également des moyens de détermination 16 d'un score de spasticité à partir de mesures de la réaction de l'articulation A à une flexion exercée sur l'articulation A du sujet, flexion exercée par le praticien. Le procédé d'utilisation du dispositif sera décrit en détail par la suite. Les moyens de détermination 16 peuvent être situés sur la première ou sur la deuxième partie du dispositif. Alternativement, les moyens de détermination peuvent être sous la forme de plusieurs parties interconnectées, chacune située sur une partie du dispositif.

Les moyens de détermination 16 comprennent un moyen de mesure 160 (non représenté) d'un couple de réaction (exercé par l'articulation A en réaction à la flexion) au niveau de axe de rotation 14. Dans le cas d'une cheville, ce moyen de mesure permet de déterminer la force d'appui de la plante du pied en réaction à la flexion exercée sur la cheville. En effet, un couple, ou « moment de force » est en mécanique un effort en rotation appliqué à un axe (ici l'axe 14) sous l'effet d'une force (en l'espèce la force d'appui de la plante de pied). Le moment par rapport à un point O d'une force F dont le point d'application est au point M est défini par :

$$\vec{\mathcal{M}}_O \;=\; \vec{OM} \wedge \vec{F}(M)$$

Dans le cas d'une cheville, les moyens de mesure 160 peuvent consister soit en un couplomètre réalisant une mesure du couple en N.m$^{-1}$ au niveau de l'axe 14 (de laquelle est déduite la valeur de la force d'appui), soit en un capteur de force actif (à titre d'exemple le XFL225D FGP Sensors®) placé dans la première partie 10 qui mesure directement la force d'appui de la plante de pied.

Ce paramètre de force, qui n'était jamais mesuré par les dispositifs connus, permet de rapprocher l'échelle qualitative d'Ashworth à la forme (amplitude et temps) des signaux obtenus.

Les moyens de détermination comprennent en outre une unité de traitement 161 (non représentée) d'un signal émis par le moyen de mesure 160 du couple de réaction de l'articulation A au niveau de l'axe de rotation 14, l'unité de traitement 161 étant destiné à déterminer :

- la composante continue dudit signal,
- la composante alternative dudit signal.

Une telle détermination des composantes continue et alternative du signal est aisément accessible par l'homme du métier, par exemple à l'aide des éléments de filtrage largement connu de l'état de la technique. A titre d'exemple, on peut mettre en oeuvre un filtrage passe-haut à 3Hz avec une amplification d'un facteur 50 pour déterminer la composante alternative.

Les moyens de détermination 16 comprennent un moyen de mesure 162 (non représenté) de l'activité musculaire d'au moins une partie des muscles de l'articulation A. Plus précisément, le moyen de mesure 162 de l'activité musculaire d'une partie des muscles de l'articulation A comprend au moins deux électrodes E1 et E2, préférentiellement agencées au niveau du rebord 1222 de l'extension 122 permettant d'établir un électromyogramme de l'activité musculaire de la partie haute du membre M2. Dans le cas d'une cheville, le moyen de mesure 162 permet d'établir un électromyogramme des muscles extenseurs de la cheville comme le muscle soléaire et/ou les muscles jumeaux. L'établissement d'un tel électromyogramme à partir d'électrodes est largement connu de l'état de la technique.

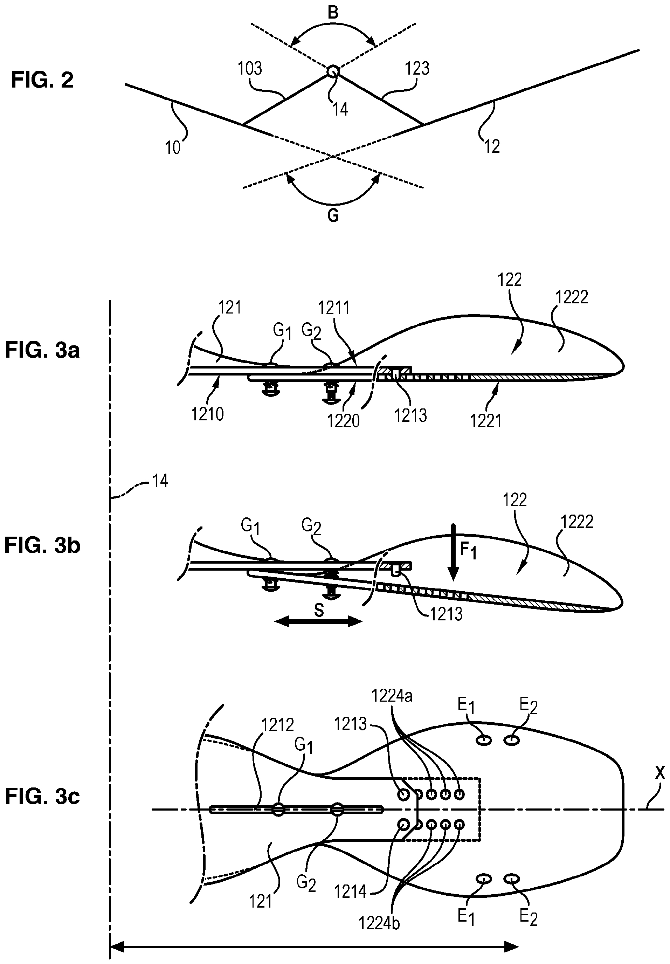

En référence à la figure 2, les moyens de détermination 16 comprennent également un moyen de mesure 163 (non représenté) de l'angle G entre la première partie 10 et la deuxième partie 12. Toutefois, la seule mesure qui est accessible directement, c'est la mesure de l'angle β formé entre les deux membres de charnière 103 et 123 du dispositif au niveau de l'axe de rotation 14. En référence à la figure 2 représentant schématiquement le dispositif, le membre de charnière 103 présente avec la surface 100 de la première partie 10 un angle $\alpha_1$. Le membre de charnière 123 présente avec la surface 1211 de la deuxième partie 12 un angle $\alpha_2$. En conséquence, afin d'obtenir la valeur de l'angle G que présente les surfaces 100 et 1211 (respectivement de la première 10 et de la deuxième partie 12) en fonction de l'angle β mesuré au niveau de l'axe de rotation 14, il suffit d'appliquer la relation suivante :

$$G = \alpha_1 + \alpha_2 - \beta$$

$\alpha_1$ et $\alpha_2$ étant fixes, un simple tarage suffit pour déterminer l'angle G à partir de la mesure de l'angle β et vice versa, β étant l'image de G à une constante près.

Ainsi les moyens de détermination 16 récoltent les informations mesurées suivantes :

- la composante continue du couple de réaction de l'articulation A au niveau de l'axe de rotation 14,
- la composante alternative du couple de réaction de l'articulation A au niveau de l'axe de rotation 14,
- l'activité musculaire d'une partie des muscles de l'articulation A,
- l'angle G formé entre les deux parties 10 et 12 du dispositif au niveau de l'axe de rotation 14.

A partir de ces informations, les moyens de détermination 16 déterminent un score de spasticité.

Le calcul d'un score est préférentiellement fondé sur une normalisation en temps de la manoeuvre, sur une intégration du signal de force, une cotation selon l'angulation et la vitesse. Préférentiellement, le score global est déterminé par une combinaison linéaire des quatre informations.

Avantageusement les moyens de détermination 16 comprennent une unité de calcul (comme un microcontrôleur) et un moyen de transmission 164 de données (non représenté), préférentiellement un moyen de transmission sans fil, par exemple par protocole WiFi ou Bluetooth à un dispositif de réception distant (non représenté), tel qu'un ordinateur, permettant ainsi d'enregistrer les informations mesurées et/ou le score de spasticité.

Alternativement, le score de spasticité est déterminé par le dispositif de réception distant à partir des informations mesurées transmises. Avantageusement, le score est affiché par l'ordinateur ou par un dispositif d'affichage localisé sur le dispositif, tel qu'un écran LCD, à destination du praticien (l'affichage étant d'une taille suffisante pour que le praticien puisse lire aisément l'information affichée). Alternativement, le score est transmis au praticien sous forme d'un son (diction du score, émission d'une tonalité plus ou moins aigu et/ou forte selon le score) émis par un élément de haut parleur agencé sur le dispositif. A partir de ce score, le praticien peut établir un diagnostique concernant la spasticité du suj et et éventuellement proposer un traitement à administrer.

Préférentiellement le dispositif présente une symétrie selon son axe longitudinal X. Ainsi un même dispositif convient aussi bien à une articulation gauche qu'à une articulation droite. De plus le dispositif est manipulable par un praticien droitier ou gaucher, des éléments de transmission du score au praticien étant pourvus sur chacun des cotés du dispositif ou étant déplaçables pour être visibles de chacun des cotés du dispositif.

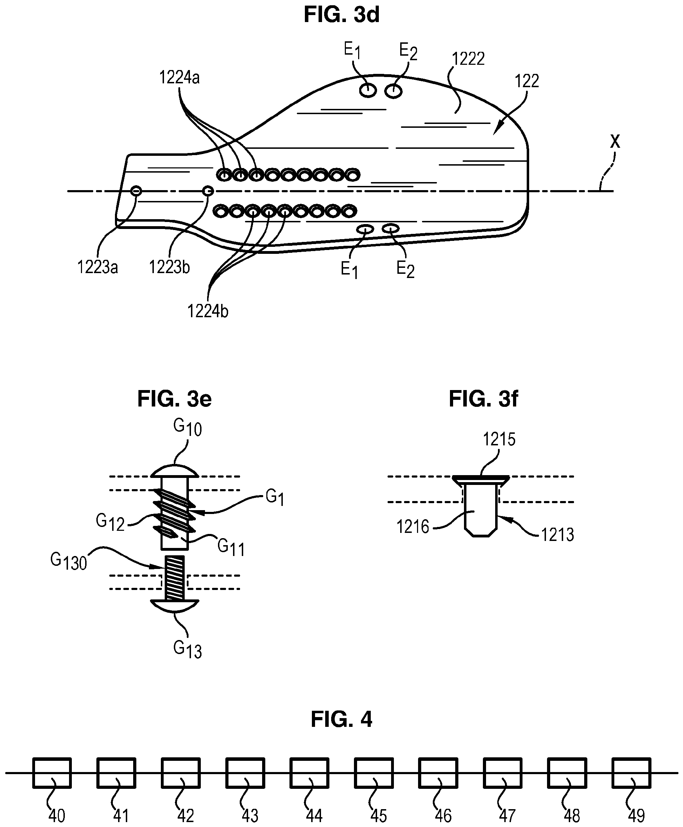

En référence aux figures 3a à 3d, la plaque 121 comprend une face supérieure 1211 destinée à recevoir une partie basse du membre M2 et une surface inférieure 1210 destinée à être en contact avec l'extension 122. La plaque 121 comprend, sur sa face 1211 une fente débouchant oblongue 1212 disposée sur la médiane le long de l'axe principal X de la plaque 121. Sur la surface inférieure 1210, au niveau d'une extrémité de la plaque 121 sont disposés deux ergots 1213 et 1214 protubérant.

L'extension 122 comprend une face 1221 destinée à recevoir une partie haute du membre M2 et à être en contact avec la face inférieure 1210 de la plaque 121. L'extension 122 comprend deux trous débouchant principaux 1223a et 1223b disposés sur la médiane le long de l'axe principal X de l'extension 122 de telle sorte qu'une fois mis en correspondance l'extension 122 et la plaque 121, les trous principaux 1223a et 1223b sont alignés verticalement avec la fente 1212 de la plaque 121. L'extension 122 comprend également deux séries de trous débouchant secondaires 1224a et 1224b situées de part et d'autres de la médiane le long de l'axe principal X de l'extension 122. Chaque série de trous débouchant est alignée parallèlement à l'axe principal X de l'extension 122.

La plaque 121 et l'extension 122 sont reliées de telle manière que les trous principaux 1223a et 1223b de l'extension 122 sont alignés verticalement avec la fente 1212 de la plaque 121. Deux éléments de liaison G1 et G2 sont insérés dans les trous débouchant principaux 1223a et 1223b afin de servir d'éléments de guidage et d'assurer ainsi une liaison glissière entre la plaque 121 et l'extension 122 le long de l'axe principal X.

En référence à la figure 3e, chaque élément de liaison G1 et G2 comprend une butée supérieure G10 destinée à venir en contact avec la surface supérieur 1211 de la plaque 121, la butée G10 étant préférentiellement cylindrique de section circulaire d'un diamètre supérieur à la largeur de la fente 1212 afin de pouvoir servir de butée. Chaque élément de liaison G1 et G2 comprend également une tige G11 reliée à l'élément de butée G10, le diamètre de ladite tige étant inférieur à la largeur de la fente 1212 et au diamètre des trous principaux 1223a et 1223b. Ainsi la tige traverse la fente 1210 et les trous principaux 1223a et 1223b. Chaque élément de liaison G1 et G2 comprend également un élément résilient tel qu'un ressort G12 associé à un butée inférieure G13. Préférentiellement, cette butée inférieure G13 est séparable du reste de l'élément de liaison et est fixée par vissage d'une partie filetée G130 de la butée inférieure G13 dans un trou taraudé au sein de la tige G11. L'élément résilient est destiné à être en contact avec la surface inférieure 1220 de l'extension 122 de sorte que les éléments de liaison G1 et G2 exerce une pression entre la plaque 121 et l'extension 122 dans le sens de la mise en contact de la surface 1210 de la plaque 121 et la surface 1221 de l'extension 122.

En référence à la figure 3f, l'ergot 1213 (similaire à l'ergot 1214) comprend une partie de butée 1215 destinée à venir en contact avec la surface 1211 de la plaque 121 et un corps 1216 destiné à venir se loger dans un des trous des deux séries de trous débouchant secondaires 1224a et 1224b. Préférentiellement, l'ergot 1213 est fixé par serrage dans un tour de la plaque 121 prévu pour recevoir l'ergot.

Une fois les deux surfaces 1210 et 1221 mises en contact les ergots 1214 et 1213 (représentés en transparence sur la figure 3c) sont respectivement insérés dans l'un des trous des deux séries de trous débouchant secondaires 1224a et 1224b permettant ainsi de bloquer en translation la plaque 121 et l'extension 122.

Ainsi la distance D entre les deux électrodes E1 et E2 et l'axe de rotation 14 est réglable. En effet, pour modifier la distance D, le praticien exerce une pression $F_1$ sur l'extension 122 à l'encontre de la pression exercée par les éléments de liaison G1 et G2 afin de dégager les ergots 1213 et 1214 des trous débouchant secondaires 1224a et 1224b comme représenté à la figure 3b. Une fois les ergots dégagés, la liaison glissière entre la plaque 121 et l'extension 122, assurée par les éléments de liaison G1 et G2 est libérée et le praticien (tout en maintenant la pression $F_1$) peut modifier la distance D en manoeuvrant l'extension 122 suivant le sens S le long de son axe principal. Une fois la distance choisie, le praticien relâche la pression $F_1$ qu'il exerçait et la pression des éléments de liaison G1 et G2 permet une remise en contact de la plaque 121 et de l'extension 122 et une insertion des ergots 1213 et 1234 respectivement dans un des trous des séries de trous débouchant secondaires 1224a et 1224b permettant ainsi de bloquer en translation la plaque 121 et l'extension 122 (comme représenté à la figure 3a).

Un tel agencement permet de placer correctement les électrodes E1 et E2 par rapport au membre M2 de l'articulation A. Le praticien place correctement les électrodes au niveau du ou des muscles à accueillir au niveau de la deuxième partie 12 du dispositif et ainsi de garantir un électromyogramme de bonne qualité de l'activité musculaire du membre M2.

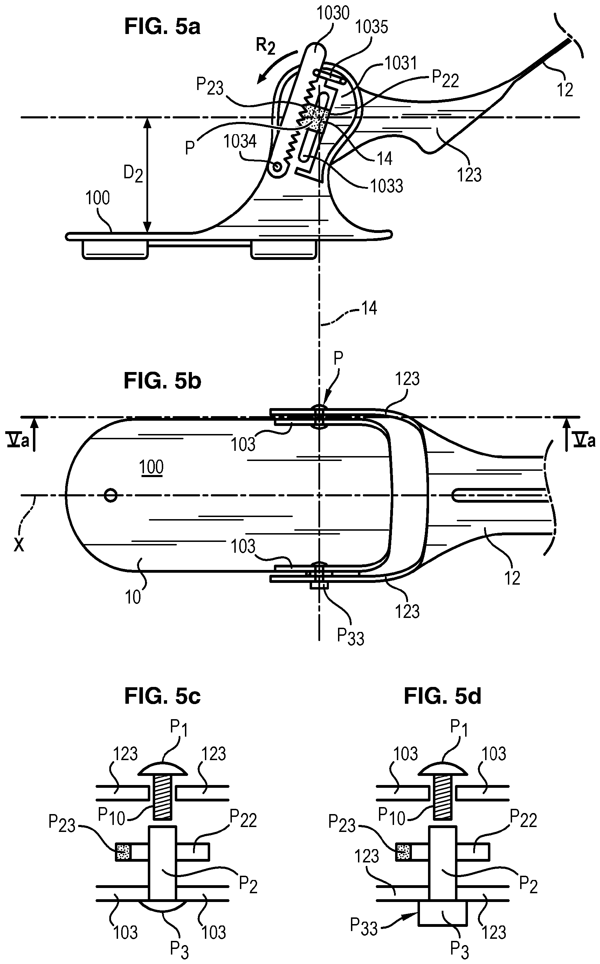

En référence aux figures 5a et 5b, la distance $D_2$ entre la surface 100 de la première partie 10 du dispositif et l'axe 14 est réglable. A cet effet, le membre de charnière 103 de la première partie 10 comprend une fente 1033 s'étendant transversalement à la surface 100 de la première partie 10 et préférentiellement perpendiculairement à l'axe 14. Un élément de crémaillère 1030 est fixé en pivot autour d'un élément pivot 1034 (et parallèlement à l'axe 14) au membre de charnière 103 de sorte que la denture de l'élément de crémaillère 1030 soit parallèle à la fente débouchante 1033. Le membre de charnière 123 de la partie 12 est relié au membre de charnière 103 par un élément de liaison P.

En référence à la figure 5c, l'élément de liaison P comprend une butée P1 et une tige P2, le diamètre de la tige étant de diamètre inférieur à la largeur de la fente 1033 et à un trou débouchant du membre de charnière 123 de la partie 12 dans lequel l'élément de liaison P est inséré. L'élément de liaison P comprend un élément de buté P3. Ainsi les butées P1 et P3 sont destinées à venir en contact avec les membres de charnière 103 et 123. La tige P2 de l'élément de liaison P comprend en outre une partie centrale P22 comprenant une partie de denture P23 destinée à venir en coopération avec l'élément de crémaillère 1030. Afin de maintenir la partie de denture P23 en coopération avec l'élément de crémaillère 1030, un élément de maintien 1031 est prévu, permettant de maintenir la partie centrale P22 en position verrouillée. Préférentiellement l'élément de crémaillère 1030, l'élément de maintien 1031 et la partie centrale P22 sont tous trois situés entre le membre de charnière 103 et le membre de charnière 123. Le maintien est par exemple réalisé par une pression exercée par un ressort 1035 de rappel relié entre l'élément de crémaillère 1030 et le membre de charnière 103 permettant d'exercer une pression sur l'élément de crémaillère 1030 en direction de l'élément de maintien 1031. Une fois en position verrouillée, la denture P23 est maintenue en position par l'élément de crémaillère 1030 et l'élément de liaison P est en conséquence maintenu en position dans la fente 1030. L'élément de liaison P étant en liaison pivot avec le membre d'articulation 123 de la deuxième partie 12, une fois l'ensemble verrouillé, la deuxième partie 12 n'est plus qu'en liaison pivot avec la première partie 10. Lorsque l'ensemble est déverrouillé (en dégageant l'élément de crémaillère 1030 en allant à l'encontre la force du ressort de rappel 1035, suivant le sens R2), l'élément de liaison P est en circulation libre le long de la fente 1030.

Ainsi, lorsque le praticien veut régler la distance D2, il déverrouille l'élément de crémaillère 1030, ce qui permet de libérer la partie de denture P23. L'élément de liaison P est donc de nouveau libre de glisser le long de la fente 1033. Une fois la bonne position choisie, le praticien verrouille l'élément crémaillère 1030 ce qui maintient l'élément de liaison P en position dans la fente 1033 à l'aide du ressort de rappel exerçant une pression sur l'élément de crémaillère. En conséquence, le praticien peut modifier la distance D2 séparant la surface 100 de l'axe de rotation 14 afin d'adapter le dispositif à l'articulation A qu'il doit accueillir. Préférentiellement, la butée supérieure P1 est séparable du reste de l'élément de liaison P et est fixée par vissage d'une partie filetée P10 de la butée supérieure P1 dans un trou taraudé au sein de la tige P2.

Préférentiellement, la liaison tel que décrite est réalisée en deux parties symétriques agencée de part et d'autre de l'axe principal X comme représenté à la figure 5b. En référence à la figure 5e (et comme représenté aussi à la figure 5b), au moins un des deux éléments de liaison P comprend un capteur angulaire P33 situé en butée et permettant de mesurer la position angulaire d'un membre de charnière par rapport à l'autre (comme expliqué précédemment).

En référence à la figure 4, elle concerne un procédé d'utilisation du dispositif de l'invention comprenant les étapes suivantes :

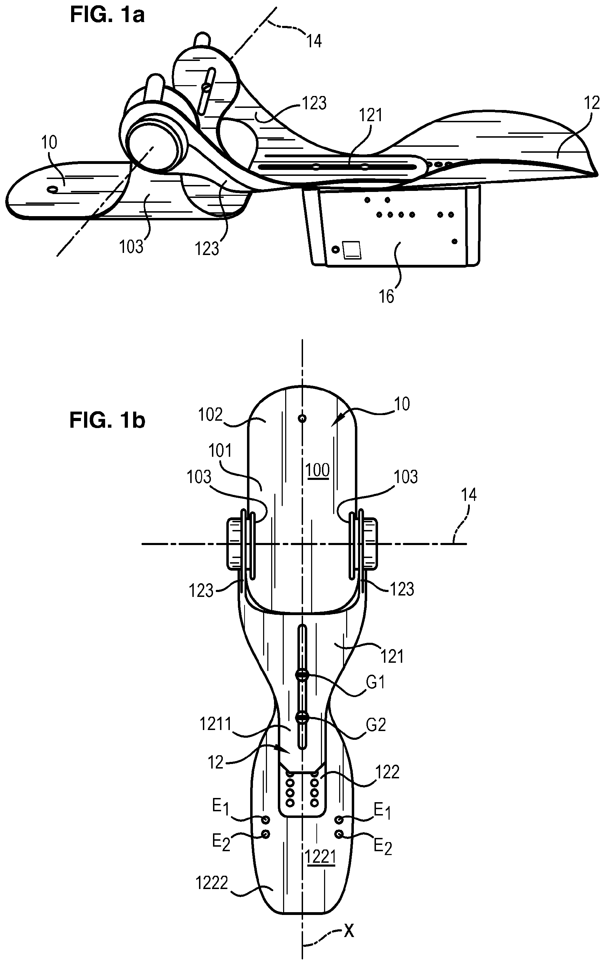

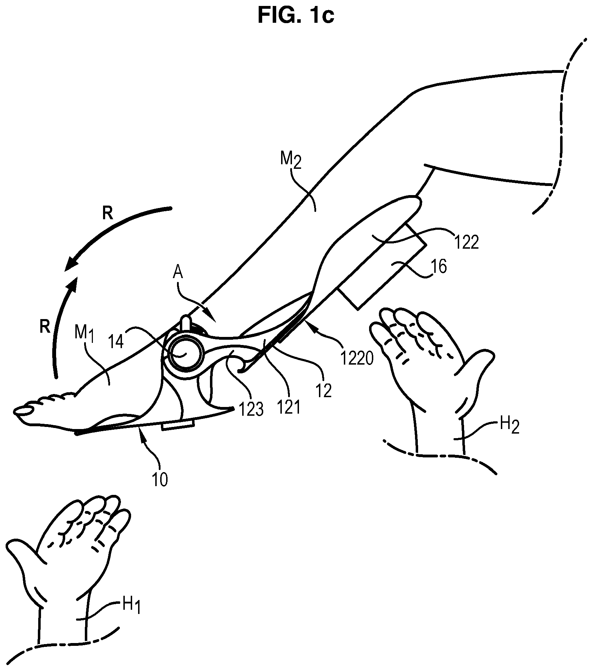

- fournir un dispositif selon l'invention (étape 40). Par exemple le praticien (médecin, kinésithérapeute, infirmier, aide-soignant, ...) peut disposer d'un tel dispositif dans son cabinet,
- ensuite, le praticien place l'articulation du sujet dont il veut mesurer la spasticité. Pour ce faire, il place le premier membre de l'articulation, par exemple le pied, au niveau de la première partie du dispositif (étape 41),
- il place également le deuxième membre de la même articulation, par exemple la partie inférieure de la jambe, au niveau de la deuxième partie du dispositif (étape 42),
- le praticien applique ensuite une flexion sur l'articulation suivant le sens R représenté à la figure 1c (étape 43). A cet effet, le praticien prend d'une main H1 la première partie 10 et de l'autre main H2 la deuxième partie 12 pour opérer une flexion de l'articulation dans le sens R comme représenté à la figure 1c,
- la réaction de ladite articulation A est alors mesurée (étape 44) tel qu'expliqué précédemment. A cet effet, il est par exemple prévu que le praticien déclenche l'acquisition des signaux par appui sur un bouton fixé sur le dispositif, par exemple sur une partie logeant les moyens d'acquisition 16). Alternativement, l'acquisition est déclenchée automatiquement dés que le dispositif présente un angle prédéterminé entre sa première et sa deuxième partie,
- les informations sont éventuellement envoyées par transmission sans fil à un dispositif de réception des données comme un ordinateur pour enregistrer les données et/ou afficher le score de spasticité (étape 45),
- le praticien attend l'arrêt des acquisitions (il est possible par exemple de sélectionner préalablement la durée des acquisitions). Alternativement, le praticien arrête l'acquisition par pression sur un bouton prévu à cet effet, qui peut être le même bouton que pour le démarrage d'acquisition (étape 46),

• détermination du score de spasticité à partir des mesures effectuées comme expliqué précédemment (étape 47),
• affichage du score de spasticité (étape 48)
• à partir des informations à sa disposition, le praticien effectue un diagnostique concernant la spasticité du sujet et d'un éventuel traitement à administrer (étape 49).

Il est à noter que le dispositif décrit a fait l'objet de deux séries d'évaluation sur des patients spastiques, à l'hôpital Saint-Jacques à Nantes.

Pour la première série d'évaluation, quatre patients spastiques ont participé aux essais de l'orthèse. Ces patients sont atteints de spasticité à divers degrés, de 1+ à 3, sur l'échelle d'Ashworth modifiée. Pour chaque patient, le dispositif a été testé après différents tests cliniques (d'une durée moyenne d'une heure) concernant leur spasticité au niveau des membres inférieurs comme l'Analyse Quantifiée de la Marche, des stimulations tendineuses et musculaires (muscle soléaire) et des exercices de mouvement de cheville.

La sollicitation articulaire et musculaire tend à provoquer une adaptation des muscles étirés. Plus le réflexe d'étirement a été sollicité, plus le sujet réagira rapidement à une manipulation d'Ashworth. Ainsi, dans le cadre de l'expérimentation, il est à noter que les quatre patients testés se trouvaient dans le même contexte car ils ont effectué les mêmes exercices au préalable.

Pour la seconde série d'évaluation, deux patients ont participé au test ; le premier, avec les mêmes conditions que celles de la première série, tandis que l'autre patient a été évalué avant les tests cliniques.

Les expérimentations ont été réalisées genoux tendu. Aucune consigne particulière n'a été donnée au praticien concernant la manipulation de l'orthèse. Le muscle soléaire a été choisi pour la mesure de l'activité électrique (EMG).

En résultat, lors des manoeuvres réalisées, les praticiens n'ont exprimé aucune gène et ont pu scorer les patients. Le ressenti avec ou sans l'orthèse donne le même score pour le même patient.

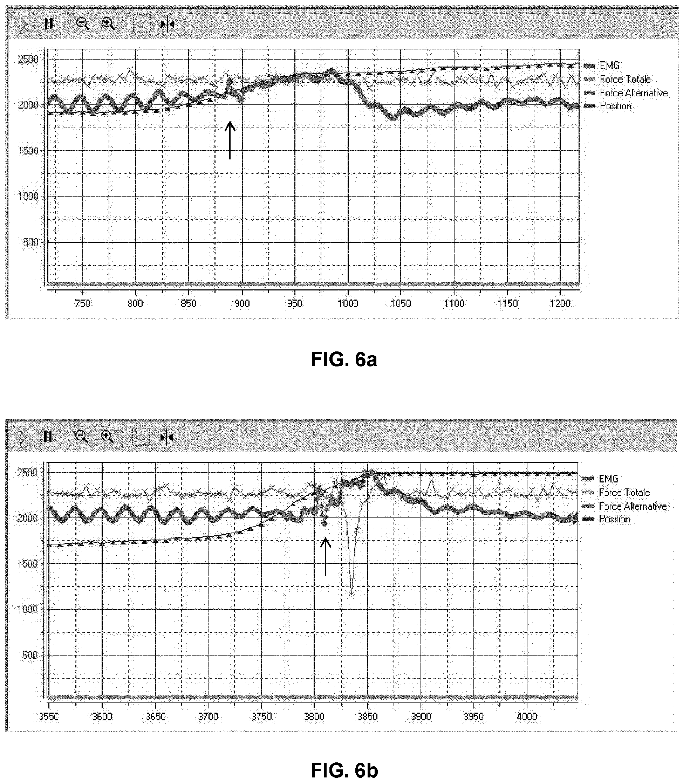

Les résultats obtenus sur les patients testés sont sous forme de résultats graphiques (figures 6a-c) et de résultats numériques.

Sous forme graphique, les variations temporelles des signaux acquis montrent que l'ensemble des patients testés ont une atteinte spastique soit par l'occurrence d'un ressaut unique ou par l'occurrence d'un clonus.

La manoeuvre d'Ashworth est réalisée selon une vitesse de dorsiflexion dénommée lente ou rapide par le praticien. Est appelée vitesse lente, une vitesse instantanée estimée entre 0.05 et 0.09°/ms (n=ll) et est appelée vitesse rapide, une vitesse instantanée estimée entre 0.12 et 0.6 °/ms (n=9). Les figures 6a et 6b représentent les signaux bruts obtenus pour le Patient 2 (niveau d'atteinte 1+) lors d'une manoeuvre lente (fig 6a) et rapide (fig 6b).

Les 4 signaux bruts (figure a) sont la force totale (FT), la force alternative (FA), la position angulaire (Angulation ou Position) et l'électromyogramme du muscle soléaire.

Lors du test de la dorsiflexion, le ressaut de force est visible sur la composante alternative de la force (FA) comme étant la partie variable de la force totale (FT) mais aussi sur le signal EMG du soléaire (voir flèche).

Compte tenu de l'angulation de départ différente de la cheville, selon que le test est réalisé à vitesse lente ou rapide, le ressaut est déclenché pour une position angulaire différente.

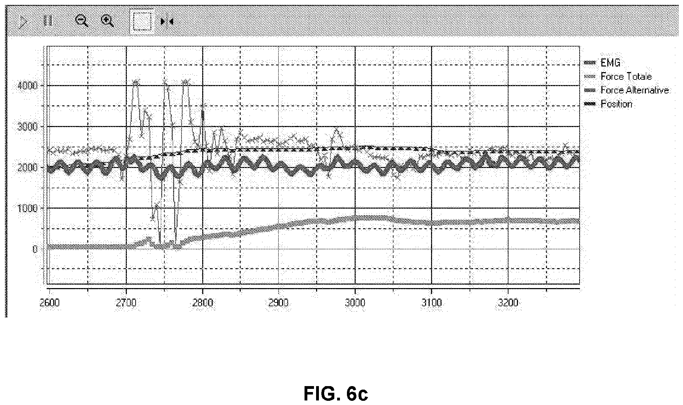

Le faible niveau de la spasticité d'un patient (1+) est en accord avec le faible niveau de force de réaction lors de la dorsiflexion (figure 6a) ; l'inverse est vrai comme le montre la figure 6c obtenue pour le Patient 4, lequel présente un niveau de spasticité de 3 sur l'échelle d'Ashworth qui comprend 5 niveaux.

On note le déclenchement d'un clonus qui est épuisable et une réaction passive du patient plus importante que pour le patient précédent

Le logiciel de traitement permet de calculer la vitesse et l'accélération angulaires à partir du signal de position angulaire et aussi de filtrer les signaux.

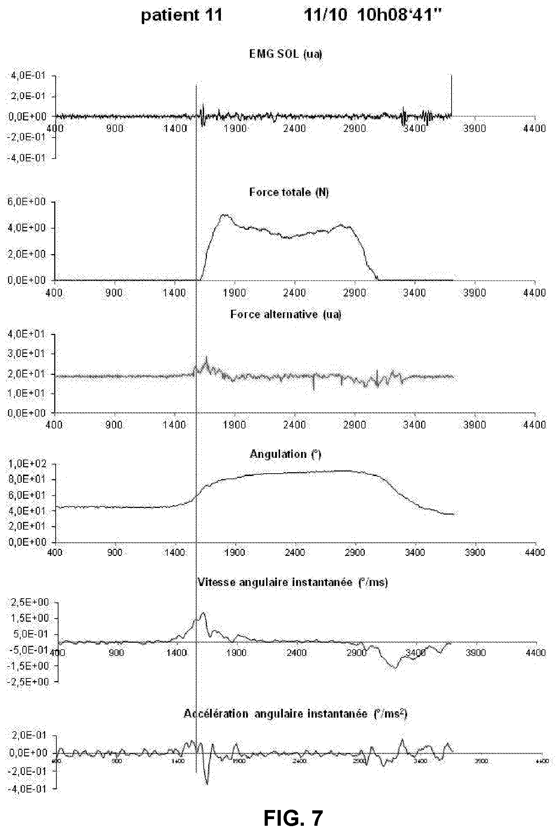

Les résultats sont exportables sur un tableur (type Excel®) et permettent d'obtenir les tracés, tels ceux du patient 11 (niveau d'atteinte 1) qui sont représentés sur la figure 7

Le ressaut ou le début d'un clonus sont identifiables au niveau des signaux de force alternative (FA) ou et de l'EMG du soléaire. Après la détermination visuelle du ressaut les valeurs d'angulation de la cheville, de vitesses moyenne et instantanée et d'accélération instantanée sont notées et reportées dans un tableau.

A titre d'exemple, pour le patient 4 précédemment mentionné (qui présentait un niveau d'atteinte de 3) on obtient le tableau suivant :

| patient 4 | AMS 3 | | | | | | |
| --- | --- | --- | --- | --- | --- | --- | --- |
| | EMG (ua) | FT (N) | FA (ua) | position angulaire (°) | Vitesse (°/ms) | vitesse moyenne (°/ms) | positions MinMax (°) |
| test1 | | | | | | | |

(suite)

| patient 4 | AMS 3 | | | | | | |
|---|---|---|---|---|---|---|---|
| | EMG | FT | FA | position angulaire | Vitesse | vitesse moyenne | positions MinMax |
| | (ua) | (N) | (ua) | (°) | (°/ms) | (°/ms) | (°) |
| T=2204 | 0,014 | 0,041 | 18,01 | 61,58 | 0,103 | 0,077 | 54,2-27,6 |
| test2 | | | | | | | |
| T=1647 | -0,003 | 0,547 | 19,01 | 67,28 | 0,173 | 0,047 | 62,2-97 |

Pour exploiter ces valeurs il est alors proposé une algorithmie utilisant les paramètres « signaux » conduisant à l'élaboration d'une échelle équivalente à celle d'Ashworth, mais adaptable également à d'autres échelles connues.

Selon l'échelle d'Ashworth modifiée,

*0 Tonus musculaire normal*

Peut être traduit par aucune activité EMG, un signal de position, de vitesse et d'accélération de la part du praticien mais en fin du mouvement de dorsiflexion une légère augmentation de la force d'appui ou « tonus » musculaire est notée comme résultant de l'étirement du groupe des extenseurs dont l'allure est comparable à l'étirement d'un ressort.

*1 Augmentation discrète du tonus musculaire se manifestant par un ressaut suivi d'un relâchement ou par une résistance minime en fin de mouvement*

Ce niveau est identifié par un ressaut en fin de mouvement accompagné par une augmentation du tonus musculaire soit une force d'appui supérieure à celle du niveau 0.
*1+ Augmentation discrète du tonus musculaire se manifestant par un ressaut suivi d'une résistance minime perçue sur moins de la moitié de l'amplitude articulaire* Ce niveau est comparable au précédent mais la résistance ou force d'appui est perçue sur moins de la moitié de l'amplitude articulaire soit après la moitié de l'angulation jusqu'à la fin du mouvement.

*2 Augmentation plus marquée du tonus musculaire touchant la majeure partie de l'amplitude articulaire, l'articulation pouvant être mobilisée facilement*

Ce niveau est comparable au précédent mais la résistance ou force d'appui est perçue lors de la dorsiflexion. Cependant la force d'appui sur l'ensemble de la dorsiflexion reste faible.

*3 Augmentation importante du tonus musculaire rendant la mobilisation passive difficile*

Ce niveau est comparable au précédent mais la résistance ou force d'appui est importante lors de la dorsiflexion.

*4 L'articulation concernée est fixée en flexion ou en extension, abduction ou adduction*

Ce niveau indique une difficulté à mobiliser l'articulation ou une force d'appui très forte. Dans ce cas la variation de l'angulation est faible pour une force d'appui importante.

Selon l'échelle de Held-Tardieu,

*0 Pas de résistance tout au long du mouvement passif*

Ce niveau est comparable au niveau 0 de l'échelle d'Ashworth

*1 Discrète augmentation de la résistance au cours du mouvement passif sans que l'on puisse ressentir clairement un ressaut à un angle précis*

Ce niveau est identifié comme étant un niveau intermédiaire entre le niveau 0 et le niveau 1 de l'échelle d'Ashworth. Seule une légère augmentation de la force est notée, sans déclenchement du ressaut.

*2 Ressaut franc interrompant le mouvement passif à un angle précis, suivi d'un relâchement*

Pour ce niveau, le ressaut est ressenti au cours du mouvement à une angulation donnée.

*3 Clonus épuisable (<10 s lorsque l'on maintient l'étirement) survenant à un angle précis*

A ce niveau, il est déclenché à une angulation donnée, un tremblement ou clonus d'une durée inférieure à 10 s.

*4 Clonus inépuisable (>10 s lorsque l'on maintient l'étirement) survenant à un angle précis*

Ce niveau est équivalent au niveau précédent mais le clonus est inépuisable c'est-à-dire qu'il a une durée supérieure à 10 s.

Selon l'échelle de cotation des réflexes à l'étirement (Buffenoir et al.) ;

*0 absent*

Pas de ressaut, à vitesse lente ou rapide.

*1 ressaut*

Existence d'un ressaut à une angulation donnée, à vitesse lente ou rapide

*2 clonus épuisable*

Déclenchement d'un clonus de courte durée à une angulation donnée, à vitesse lente ou rapide.

*3 clonus inépuisable*

Déclenchement d'un clonus de longue durée à une angulation donnée, à vitesse rapide.

*4 clonus inépuisable à vitesse lente*

Déclenchement d'un clonus de longue durée à une angulation donnée, à vitesse lente.

De par la nature quantitative précise donnée par les différents signaux, il est proposé de regrouper ces échelles de sorte à obtenir une échelle globale à 25 niveaux.

Celle-ci est fondée sur des normalisations du signal d'angulation en temps et en amplitude et de noter l'occurrence de l'augmentation de la force de réaction.

Le ressaut est identifié sur le signal EMG par une trace brève (réflexe d'étirement) précédant physiologiquement l'activité mécanique. Suite au reflexe d'étirement, une force contraire à la force d'appui est générée par le sujet.

Cette force est faible par rapport à la force d'appui ou force totale, mais est notée sur la force alternative comme une impulsion de force ; celle-ci est ressentie par le praticien comme étant un ressaut de la part du patient. Il est alors possible de la dater sur le signal d'angulation.

**Revendications**

**1.** Dispositif de mesure de la spasticité d'une articulation (A) d'un sujet, l'articulation étant située entre un premier membre (M1) et d'un deuxième membre (M2), le dispositif étant **caractérisé en ce qu'**il comprend :

    ◦ une première partie (10) destinée à recevoir tout ou partie du premier membre (M1) de l'articulation (A),
    ◦ une deuxième partie (12) destinée à recevoir tout ou partie du second membre (M2) de l'articulation (A), la deuxième partie (12) étant en liaison pivot avec la première partie (10) autour d'un axe de rotation (14), et
    ◦ des moyens de détermination (16) d'un score de spasticité à partir de mesures de la réaction de ladite articulation (A) à une flexion exercée sur l'articulation (A), les moyens de détermination (16) comprenant :

        • un moyen de mesure (160) d'un couple de réaction de l'articulation (A) au niveau de l'axe de rotation (14),
        • une unité de traitement (161) de la mesure du couple de réaction de l'articulation (A) au niveau de l'axe de rotation (14) par le moyen de mesure (160), l'unité de traitement (161) étant destinée à déterminer :

◦ la composante continue de ladite mesure,
◦ la composante alternative de ladite mesure,

• un moyen de mesure (162) de l'activité musculaire d'une partie des muscles de l'articulation (A), et
• un moyen de mesure (163) de l'angle (G) formé entre les deux parties (10, 12) du dispositif.

2. Dispositif de mesure selon la revendication 1, **caractérisé en ce que** le moyen de mesure (162) de l'activité musculaire d'une partie des muscles de l'articulation (A) comprend au moins deux électrodes (E1, E2) permettant d'établir un électromyogramme de ladite activité musculaire.

3. Dispositif de mesure selon la revendication 2, **caractérisé en ce que** la distance (D) entre les au moins deux électrodes (E1, E2) et l'axe de rotation (14) est réglable.

4. Dispositif selon l'une des revendications 1 à 3, **caractérisé en ce qu'**au moins une des première et deuxième parties (10) du dispositif comprend une surface supérieure (100) et **en ce que** la distance (D2) entre ladite surface supérieure (100) et l'axe de rotation (14) est réglable.

5. Dispositif selon l'une des revendications 1 à 4, **caractérisé en ce que** les moyens de détermination (16) comprennent en outre un moyen de transmission de données, préférentiellement un moyen de transmission sans fil.

**Patentansprüche**

1. Vorrichtung zum Messen der Spastizität eines Gelenks (A) eines Probanden, wobei sich das Gelenk zwischen einer ersten Gliedmaße (M1) und einer zweiten Gliedmaße (M2) befindet, wobei die Vorrichtung **dadurch gekennzeichnet ist, dass** sie umfasst:

o einen ersten Teil (10), der bestimmt ist, den gesamten oder einen Teil der ersten Gliedmaße (M1) des Gelenks (A) aufzunehmen,
o einen zweiten Teil (12), der bestimmt ist, den gesamten oder einen Teil der zweiten Gliedmaße (M2) des Gelenks (A) aufzunehmen, wobei der zweite Teil (12) mit dem ersten Teil (10) um eine Rotationsachse (14) in Drehgelenkverbindung ist, und
o Bestimmungsmittel (16) eines Spastizitätsscores ausgehend von Messungen der Reaktion des Gelenks (A) auf eine auf das Gelenk (A) ausgeübte Beugung, wobei die Bestimmungsmittel (16) umfassen:

• ein Messmittel (160) eines Reaktionsmoments des Gelenks (A) im Bereich der Rotationsachse (14),
• eine Verarbeitungseinheit (161) der Messung des Reaktionsmoments des Gelenks (A) im Bereich der Rotationsachse (14) durch das Messmittel (160), wobei die Verarbeitungseinheit (161) bestimmt ist zu bestimmen:

◦ den Gleichanteil der Messung,
◦ den Wechselanteil der Messung,

• ein Messmittel (162) der Muskelaktivität eines Teils der Muskeln des Gelenks (A), und
• ein Messmittel (163) des Winkels (G), gebildet zwischen den zwei Teilen (10, 12) der Vorrichtung.

2. Messvorrichtung nach Anspruch 1, **dadurch gekennzeichnet, dass** das Messmittel (162) der Muskelaktivität eines Teils der Muskeln des Gelenks (A) mindestens zwei Elektroden (E1, E2) umfasst, die erlauben, ein Elektromyogramm der Muskelaktivität zu erstellen.

3. Messvorrichtung nach Anspruch 2, **dadurch gekennzeichnet, dass** der Abstand (D) zwischen den mindestens zwei Elektroden (E1, E2) und der Rotationsachse (14) einstellbar ist.

4. Vorrichtung nach einem der Ansprüche 1 bis 3, **dadurch gekennzeichnet, dass** mindestens einer von dem ersten und zweiten Teil (10) der Vorrichtung eine obere Fläche (100) umfasst und dadurch, dass der Abstand (D2) zwischen der oberen Fläche (100) und der Rotationsachse (14) einstellbar ist.

**5.** Vorrichtung nach einem der Ansprüche 1 bis 4, **dadurch gekennzeichnet, dass** die Bestimmungsmittel (16) ferner ein Datenübertragungsmittel, vorzugsweise ein drahtloses Übertragungsmittel, umfassen.

**Claims**

**1.** Device for measuring the spasticity of an articulation (A) of a subject, the articulation being located between a first limb (M1) and a second limb (M2), the device being **characterised in that** it comprises:

    ◦ a first part (10) for receiving all or part of the first limb (M1) of the articulation (A),
    ◦ a second part (12) for receiving all or part of the second limb (M2) of the articulation (A), the second part (12) being pivotably connected to the first part (10) about a rotational axis (14), and
    ◦ means for determining (16) a spasticity score from measurements relating to the reaction of said articulation (A) to a flexion exerted on the articulation (A), the determination means (16) comprising:

        • a means for measuring (160) an articulation (A) reaction torque at the site of the rotational axis (14),
        • a unit for processing (161) the measurement of the reaction torque of the articulation (A) reaction torque at the site of the rotational axis (14) by the measuring means (160), the processing unit (161) being intended to determine:

            ◦ the continuous component of said measurement,
            ◦ the alternative component of said measurement,

        • a means for measuring (162) the muscular activity of part of the muscles of the articulation (A), and
        • a means for measuring (163) the angle (G) formed between the two parts (10, 12) of the device.

**2.** Measuring device according to claim 1, **characterised in that** the means for measuring (162) the muscular activity of part of the muscles of the articulation (A) comprises at least two electrodes (E1, E2) making it possible to establish an electromyogram of said muscular activity.

**3.** Measuring device according to claim 2, **characterised in that** the distance (D) between the at least two electrodes (E1, E2) and the rotational axis (14) is adjustable.

**4.** Device according to one of claims 1 to 3, **characterised in that** at least one of the first and second parts (10) of the device comprises an upper surface (100) and **in that** the distance (D2) between said upper surface (100) and the rotational axis (14) is adjustable.

**5.** Device according to one of claims 1 to 4, **characterised in that** the determination means (16) further comprise a data transmission means, preferentially a wireless transmission means.

**FIG. 1a**

**FIG. 1b**

# FIG. 1c

**FIG. 2**

B

103          123

14

10                    12

G

**FIG. 3a**

121        1211        122        1222

$G_1$  $G_2$

1210        1220        1213        1221

14

**FIG. 3b**

$G_1$        $G_2$        122        1222

$F_1$

S        1213

**FIG. 3c**

1212        1224a        $E_1$    $E_2$

1213

$G_1$                            X

121        $G_2$    1214

1224b        $E_1$    $E_2$

## FIG. 3d

$E_1$  $E_2$  1222  122
1224a
1223a  1223b
1224b
$E_1$  $E_2$
X

## FIG. 3e

$G_{10}$
$G_1$
$G_{12}$
$G_{11}$
$G_{130}$
$G_{13}$

## FIG. 3f

1215
1216  1213

## FIG. 4

40  41  42  43  44  45  46  47  48  49

FIG. 5a

FIG. 5b

FIG. 5c

FIG. 5d

FIG. 6a

FIG. 6b

**FIG. 6c**

FIG. 7

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2010121353 A
- US 2008071386 A1
- US 2008312549 A1
- US 2007027631 A1

**Littérature non-brevet citée dans la description**

- **ASHWORTH B.** Preliminary trial of carisoprodol in multiple sclerosis. *Practionner,* 1964, vol. 192, 540-542
- Separate Quantification of Reflex and Nonreflex Components of Spastic Hypertonia in Chronic Hemi-paresis. **CHUNG et al.** ARCHIVES OF PHYSICAL MEDICINE AND REHABILITATION, W.B. SAUNDERS. UNITED STATES, 25 Mars 2008, vol. 89, 700-710
- Quantitative measurement of spastic ankle joint stiffness using a manual device: A preliminary study. **KOBAYASHI T et al.** JOURNAL OF BIOMECHANICS. PERGAMON PRESS, 18 Juin 2010, vol. 43, 1831-1834